# EUROPEAN PATENT SPECIFICATION

(11) **EP 2 268 341 B1**
(45) Date of publication and mention of the grant of the patent: **17.06.2015**
(21) Application number: 08746455.8
(22) Date of filing: 21.04.2008
(51) Int. Cl.: A61M 5/00, A61M 35/00

(54) **ANTEGRADE COLONIC INSTILLATION APPARATUS**
ANTEGRADES KOLON-INSTILLATIONSGERÄT
APPAREIL D'INSTILLATION COLIQUE ANTÉROGRADE

(43) Date of publication of application: 05.01.2011
(73) Proprietor: Haleys Pump Company, Forestville, California 95436 (US)
(72) Inventor: MOMBRINIE, Bruno, Forestville California 95436 (US); EIDSEN, Jeff Brian, Sebastopol, CA 95472-9478 (US); EIDSEN, Cindy Carol, Sebastopol, CA 95472-9478 (US); PETERSON, Joe, Windsor California 95492 (US); DAVIS, Robert, Santa Rosa California 95409 (US)
(74) Representative: Marchitelli, Mauro
(86) International application number: PCT/US2008/061034
(87) International publication number: WO 2009/131567

(56) References cited:
- EP-A1- 0 582 032
- US-A- 3 508 546
- US-A- 4 178 931
- US-A- 4 682 979
- US-A- 4 874 363
- US-A- 5 147 293
- US-B1- 7 294 120
- US-B1- 7 294 120

## Description

The present invention relates to a human large intestine flushing apparatus such as required for patients with large intestinal disorders including fecal incontinency or intractable constipation. More particularly, to an apparatus for automatic instillation of flushing fluids to the large intestine for managing bowel movements in patients exhibiting fecal incontinency, intractable constipation or related disorders who have undergone a Malone type, Monti plasty or similar surgical procedure wherein a catheterizable stoma is constructed into the large intestine allowing insertion of the instillation apparatus for the purpose of administering an Antegrade Colonic or Continent Enema (ACE).

### BACKGROUND OF THE INVENTION

Medical disorders of the large intestine can result in symptoms which prevent the patient from adequately evacuating fecal material from the large intestine. Fecally incontinent and intractably constipated patients have difficulties managing their bowel movement. Bowel movement management is typically accomplished by a daily flushing of the large intestine by enema wherein fluids are introduced into the large intestine to flush materials retained in the intestine. Fluids can be introduced from the rectum into the large intestine to flush and allowed to drain out. There are disadvantages with the application of a rectal enema flush including the lack of penetration of fluid throughout the entire interior of the large intestine, thereby leaving fecal matter lodged in the intestine, cleanliness issues, general difficulty of self application, lack of privacy, discomfort, and bulky enema equipment.

Surgical techniques have been developed to permit the application of an enema into the large intestine from the top of the intestine as opposed to the rectum. The enema from above or Antegrade Colonic Enema requires that a patient undergo a surgical procedure to create a stoma or entry through the skin into the intestine proximal to the top of the large intestine, principally to the cecum. Fluids are introduced and permitted to flush and drain to and out the rectum. Several surgical procedures have been developed to construct an entry point intestinal stoma proximal to the cecum. The Malone or Continent Appendicostomy surgical procedure constructs a connection conduit made between the appendix and the navel (belly-button) wherein a "button" or piercing is created at the navel. Using the "button" the patient can insert a needle or catheter and deliver fluid, a process known as instillation, into the large intestine as an alternative to performing a rectal enema. The procedure permits use of the appendix or neoappendix to be used as a way to administer an antegrade colonic enema or an antegrade continent enema without a rectal maneuver.

Another similar procedure known as a Monti plasty procedure surgically fashions a conduit between the large intestine and an insertion stoma in the skin also for purposes of instillation of an antegrade colonic enema.

Regardless of the technique elected to create a stoma entry into the large intestine, the instillation of fluids requires an external mechanism to introduce the fluids into the stoma and therefore into the intestine. Fluid is typically introduced to sweep the large intestine of fecal matter at least once a day.

The typical method for irrigating patients who have undergone a Malone, Monti or similar surgical procedure have been required to introduce fluids into the large intestine by means of a drip consisting typically of a saline filled bag elevated above the patient with a drain tube attached at one end to the bag and terminated by a needle or catheter at the other end which, in turn, is inserted into the button or piercing at the navel. Fluid is permitted to drip into the large intestine which migrates to the rectum and the flushing is accomplished. The drip procedure requires the patient to remain immobile and sitting on a commode as the fluid passes through the intestine and out the rectum for a period of several hours. The procedure typically requires on standard drip bag to be used.

More recently, flushing of the intestine through the entry stoma piercing has been accomplished by introducing fluids into the large intestine by utilizing a series of fluid-filled syringes. A large syringe filled with the flushing fluid is attached to a needle or catheter and inserted into the piercing or button. The fluid contents of the syringe is dispensed and then refilled periodically until the required amount of fluid is delivered. The syringe technique requires two competent people to administer. One caretaker is required to fill the syringe. At the same time the patient is responsible for pinching the catheter shut when the syringe is removed from the catheter as introduction of flushing fluid into the intestine produces back pressure. The catheter must be pinched shut to prevent fecal matter and intestinal fluid from flowing back through the catheter and out the now open catheter end when the syringe is removed for filling. The procedure using the syringe technique requires the syringe to be refilled 10 to 20 times. If the client is not competent or able enough to assist, a second caretaker is needed. Many individuals requiring this type of treatment often have other handicaps that prevent them from assisting themselves. The use of multiple syringes also increases the risk of introducing air into the installation fluid. The introduction of quantities of air into the soma necessarily induces significant discomfort or pain in the patient.

Automated instillation devices directed to address the disadvantages of the drip bag and syringe techniques exhibit the principle disadvantage associated with the difficulty to manipulate the various elements of the device as required during self-administration, usually requiring the use of both hands for manipulation. Devices with separate fluid reservoir bags must be separated from the apparatus in preparation to fill the reservoir bag a maneuver that requires dexterity not always present in patients requiring the apparatus. Further, separation of the fluid reservoir can introduce volumes of air into the pump potentially causing discomfort to the patient during the administering the procedure.

In order to determine when a predetermined volume of instillation flushing fluid has been delivered, a pump control system may monitor the pump motor current and stop the pump motor with the pump motor current draw increase as a sealed collapsible fluid reservoir is depleted of fluid. The disadvantage of this technique is that current draw from the pump varies between pumps, the viscosity of flushing fluid, and the varying fluid resistance resulting from using differing sizes of catheters, thereby resulting in difficulties starting the apparatus and causing the pump to run dry. Alternatively the delivery volume may be determined when using an open reservoir by incorporating a level sensor to stop the flow at a predetermined volume. Such level sensors often prone to triggering falsely due to obstructions, other external effects or corrosion. In the case of open reservoir systems, level sensors located within the reservoir typically indicate an empty reservoir before the fluid is fully depleted resulting in inaccurate fluid volume delivery and residual fluid at the bottom of the reservoir complicating use of a portable device.

There is a need for an improved instillation apparatus and method to administer an antegrade colonic that avoids these disadvantages. The present invention fulfills this need, and further provides related advantages An instillation apparatus according to the prior art is for instance disclosed in document US 3 508 546 A.

### SUMMARY OF THE INVENTION

Accordingly, the present invention is directed to colonic instillation pumps and, more specifically, to such an automated apparatus with improved portability, reliability and user convenience.

The present invention further improves the portability, reliability and ease of use of prior art devices by integrating all pump components into a single unit and incorporating high reliability fluid delivery shutoff and improved volumetric delivery. The present invention therefore generally comprises a fluid reservoir having an open top and a bottom suitable for holding instillation fluid, an internal component chamber having a top and a bottom wherein the chamber top is formed from the fluid reservoir bottom being a bulkhead plate, a pump assembly having a motor, drive transfer, and manifold with the manifold mounted in the bottom of the fluid reservoir and inside the internal component chamber having an inlet and an outlet with the inlet in direct fluid communication with the fluid reservoir and the outlet protruding upwardly through the bulkhead into the fluid reservoir but not in fluid communication with the reservoir, a delivery tube being flexible and having proximate and distal ends with the proximate end in direct fluid communication with the pump manifold outlet, a catheter suitable for insertion into a patient with an intestinal stoma constructed for purposes of instilling fluid into a patient's intestines in direct fluid communication and removably attached to the distal end of the delivery tube, a pump control module communicatively attached to the pump assembly for purposes of activation and mounted in the internal component chamber, a fluid recess chamber formed in the inlet of the pump manifold and in direct fluid communication with the fluid reservoir, the fluid recess chamber being a cylindrical expansion of the pump manifold inlet has a fine sieve filter positioned level with or below the fluid reservoir, an optical level sensor mounted in the fluid recess chamber wall operable to communicate the presence of fluid in the fluid recess chamber to the pump control module to which it is communicatively attached, and a vertical draw tube forming an extension of the pump manifold inlet with the top positioned near and below the filter having a drain hole tangent with the bottom of the recess chamber, the pump control module further being in direct electrical communication with a power supply, and a user interface panel mounted proximate to the internal component chamber being in direct electrical communication with the pump control module.

The method of using the present invention to self-administer an antegrade colonic enema comprises the steps of; filling the reservoir with a flushing fluid, priming the device, inserting the catheter suitable for insertion into a patient with an intestinal stoma, activating the apparatus, waiting for the apparatus to automatically deactivate, and then removing the catheter from the patient's intestinal stoma. The operation is therefore simple and within the capabilities of most patients, including those with disabilities.

Reduced manufacturing costs are accomplished by eliminating the need for molds to be produced as the pump body may be fabricated from stock sizes of polymer tubing cut to form a cylinder. The bulkhead plate, preferably also constructed of a polymer, is pressed into place inside the polymer tube and secured in place by an o-ring circumferentially mounted to the bulkhead plate. The region above the bulkhead plate is the fluid reservoir. The region below the bulkhead plate is a component chamber wherein the bottom of the chamber comprises a circular base element also with a circumferentially mounted o-ring pressed into the bottom of the polymer cylinder.

As the apparatus is intended to be portable, in normal use the pump is periodically subjected to inspection such as at airports and other such security points, another objective of the invention is therefore to minimize the burden of security inspection procedures. Use of a transparent polymer cylinder to form the body of the apparatus enables convenient visual inspection of the device's internal components thereby improving ease of transit. Further a flexible delivery tube, being connected within the bottom of the fluid reservoir permits easy storage of the tube within the fluid reservoir for transport. Most powered instillation devices require mains power. In the present apparatus, power is supplied by batteries therefore eliminating the need for mains power to be available improving convenience, safety and regulatory acceptance.

Of noteworthy import, the present invention utilizes a unique integrated pump manifold assembly. Cleanliness of the fluid flow paths in devices that deliver fluid to the body is important. Keys to maintaining the cleanliness of the pathway include reducing the overall path length, minimizing back flow, and to reduce the number of crevices or surface imperfections where debris may collect. Peristaltic pumps, well know to those in the field, are typically used in the prior art because the peristaltic pumps do not require a back flow restriction device and the activating surfaces of the pump are comprised of a flexible tube, known as a tube set, which is squeezed. The tube set has a smooth interior and requires replacement. Unfortunately peristaltic pumps are large and bulky compared to gear pumps with similar volume capabilities. And, repeated squeezing of the tube set eventually results in sloughing of material from the tube into the delivered fluid as the tube wears. Consequently, the tube set must be replaced frequently. Peristaltic pumps also consume more power than gear pumps. The characteristics of peristaltic pumps are therefore not suitable for small and portable instillation pumps. The pump manifold assembly of the present invention overcomes these disadvantages by incorporating a gear pump in combination with a back flow restrictor that permits the use of a gear pump. The back flow restrictor is located in the fluid path of the manifold and prevents back flow into the delivery tube and the reservoir through the gear pump. Further the restrictor requires a small fluid pressure in order to allow a forward flow thereby assuring firm seating of a valve ball on the valve seat in the restrictor and hence a secure seal. Still further by integrating the fluid flow direction restrictor in-line and in close proximity to the gear pump, the fluid path can be constructed in such a fashion that it is short and having a smooth interior while gaining the advantages of the reliability of gear pumps.

Typical back flow restrictors utilize a diaphragm construction that tend to allow back flow at low pressures and also provide surface areas where deposits can collect. The smooth surface of the valve ball and small valve seat surface area mitigate the buildup problem and prolong the life of the mechanism. A smooth surfaced glass material is used form the valve ball in the present invention.

Water and flushing fluid intrusion into the interior of mechanized instillation devices manifests undesirable corrosion and deterioration of internal components thereby reducing the mean time to failure of the pump and the various electronic control components. The construction of the present invention utilizing a sealed interior component chamber increases reliability and availability of the apparatus by attaining improved water resistance over prior devices. Hence, a further objective of the invention is to provide an instillation pump that is water resistant for protection of the internal components and for more convenient cleaning.

Ideally, the instillation fluid draw from the fluid reservoir should be complete such that no or minimal draining by the user is required. To accomplish this objective, the present invention incorporates a fluid recess chamber in the inlet of the pump manifold presenting a fine sieve filter at a low point within the bottom of the fluid reservoir through which the instillation flows into the chamber having a vertical draw tube in direct contact with inlet portion of the pump at the bottom of the chamber, with the top end open near the sieve filter and a small opening on the side of the tube near the bottom of the recess chamber. An optical fluid level sensor is positioned to face into the fluid recess chamber and is used to detect low fluid level and, in turn, is in direct electrical connection with the pump control module for purposes of shutting off the pump motor when the fluid level is low. The fluid recess chamber mechanism facilitates the use of a fine sieve filter which, due to the surface tension of the instillation fluid may retain a small air bubble or bubbles below the filter in normal use. This condition also occurs during initial filling of the reservoir as air is trapped below the filter. A vertical draw tube top opening is positioned such that when the pump is started, any small air bubble is drawn away from the filter thereby preventing trapped air which can prevent fluid from entering the fluid recess chamber or falsely indicate an empty reservoir condition. The small opening in the side of the vertical draw tube then also permits instillation fluid to drain from the recess chamber and below the optical fluid level sensor. The pump control module further orchestrates a sequence of steps when the apparatus is first activated to manage the air removal from the recess chamber.

### BRIEF DESCRIPTION OF THE DRAWINGS

The accompanying drawings, which are incorporated in and constitute a part of this specification illustrate embodiments of the invention and, together with the description, serve to explain the features, advantages, and principles of the invention.

In the drawings:
FIGURE 1 is a perspective view of an embodiment of the portable instillation apparatus constructed in accordance with this invention when arranged so as to illustrate the major components of the pump assembly of the invention.
FIGURE 2 is a top plan view of the invention shown in Figure 1 showing the inlet and outlet of the pump manifold in the bottom of the fluid reservoir.
FIGURE 3 is a bottom plan view according to the present invention showing the removable battery compartment cover.
FIGURE 4 is an elevation sectional view taken on Line 4-4 of Figure 2 showing details of the pump manifold according to the present invention.
FIGURE 5 is an enlarged elevation sectional view of the portion at 5 of Figure 2 showing details of the fluid recess chamber with instillation fluid present in the fluid reservoir.
FIGURE 6 is similar to Figure 5 showing details of the fluid recess chamber with an empty fluid reservoir.

### DETAILED DESCRIPTION OF THE PREFERRED EMBODIMENT

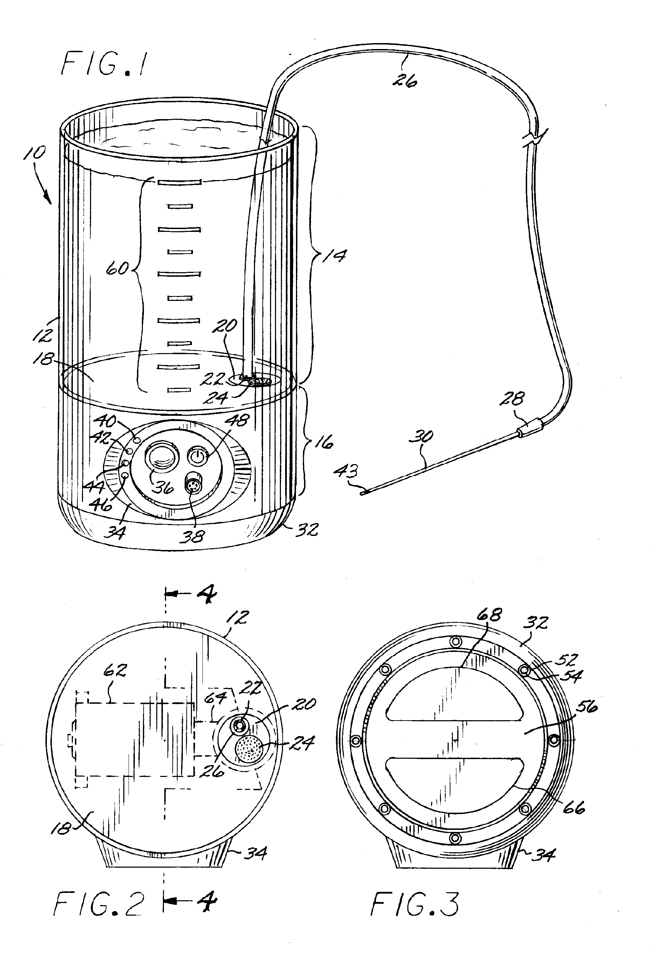

Although particular embodiments of the invention have been described in detail for purposes of illustration, various modifications may be made without departing from the spirit and scope of the invention. Accordingly, the invention is not to be limited except as by the appended claims. Referring now in greater detail to the various figures of the drawing wherein like reference characters refer to like parts, there is shown at 10 in Fig 1, portable instillation apparatus constructed in accordance with the subject invention. It will be appreciated that while the apparatus is particularly suited for affecting an antegrade colonic enema procedure, device may also be used of other similar irrigations of the intestines as well. Moreover, the subject invention can further be used for non-enema applications wherein instillation of a fluid is indicated.

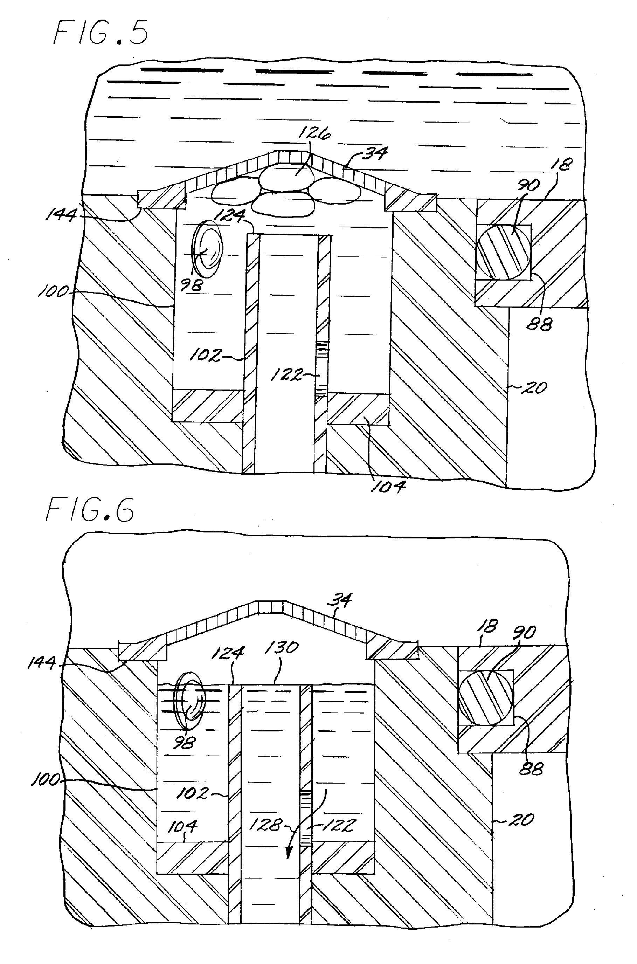

A preferred embodiment of the instant invention is illustrated in Figures 1 - 6. Figures 5-6 are directed to details of the fluid recess chamber, an essential element of the present invention.

In the preferred embodiment, the present invention comprises a cylindrically shaped tube forming the housing for a fluid reservoir having a top and a bottom, a circular bulkhead plate having a top and a bottom, forming the bottom of the fluid reservoir and the top of a component chamber, pressed and sealed, by means of an o-ring, into the inside circumference of the cylinder housing, the component chamber having a top and a bottom with bottom being a circular base inserted into the inside circumference of the bottom of the cylinder housing, sealed by means of an o-ring, thereby enclosing and protecting the component chamber from fluid intrusion, the circular base further comprising a battery cavity accessible from the bottom of the base by removing a battery cover threaded to the circular base suitable for retaining a battery, a pump manifold mounted through the bottom of the bulkhead pate having an inlet and an outlet with the inlet in direct fluid communication with the fluid reservoir and the outlet protruding upwardly from the pump manifold into the fluid reservoir but not in fluid communication with the reservoir, the pump manifold further comprising a gear pump having a pump motor, the gear pump being in direct fluid communication with the manifold inlet and a back flow restrictor, the restrictor being operable to permit flow only in a direction from the manifold inlet, the restrictor being in further direct fluid communication with the manifold outlet, a delivery tube being flexible and having a proximate and distal end with the proximate end in direct fluid communication with the pump manifold outlet, a catheter suitable for insertion into a patient with an intestinal stoma constructed for purposes of instilling fluid into a patient's intestines in direct fluid communication and removably attached to the distal end of the delivery tube, a pump control module communicatively attached to said gear pump motor suitable for activating the pump motor, the manifold inlet further comprising a fluid recess chamber being a cylindrical expansion of the inlet comprising a fine sieve filter fitted to the top of the recess chamber and in direct fluid communication with the reservoir, a vertical draw tube with a top near and below the filter being removably inserted into the pump manifold inlet and secured with a seal, and an optical fluid level sensor mounted in wall of the recess chamber operable to communicate the presence of fluid to the pump control module, the pump control module further being in direct electrical communication with the battery, a user control panel mounted to the cylinder proximate to the component chamber with a switch and indicators being in direct electrical communication with the pump control module, and a lid assembly being generally circular is detachably inserted into the circumference of the top of the fluid reservoir.

Referring to Figure 1 at 10, an overall perspective view and the preferred embodiment of the apparatus according to the present invention, the portable instillation apparatus is of a unibody cylindrical construction comprising a transparent polymer cylinder 12 forming the housing wherein a fluid holding portion forms the reservoir 14 and a component portion forms the component chamber 16. The fluid reservoir portion is defined by the inside of the polymer cylinder 12 and the bulkhead 18 forming the bottom of the reservoir 14 which is pressed into the inside circumference of the polymer cylinder 12. The pump manifold assembly 20 has a circularly shaped top pressed into the bulkhead 18 from below into a circularly shaped receiving opening in the bulkhead 18. An pump manifold 20 further has outlet adapter 22 directed upwardly into the fluid reservoir 14 for adapting the outlet of the pump manifold 20 to a delivery tube 26 and providing direct fluid communication from the outlet of the pump manifold 20 to the deliver tube 26 without providing for fluid communication with the fluid reservoir 14. A fine sieve filter 24 is removably pressed into the inlet of the pump manifold 20 through which fluid is drawn being secured by a resilient draw tube seal 104 and being in direct fluid communication with the fluid reservoir 14, thereby prohibiting particulate matter from entering the pump manifold 20.

Still referring to Figure 1, a flexible delivery tube 26 is removably pressed onto the pump manifold outlet adapter 22 at the proximate end of the tube while the distal end is removably pressed into a catheter adapter 28 having an inlet sized to receive the flexible delivery tube 26 and an outlet to receive a stoma catheter 30. A stoma catheter 30 is removably pressed into the outlet of the catheter adapter 28 wherein the assembled components provide for direct fluid communication from the pump manifold outlet to and through the stoma catheter delivery openings 43 and into a stoma on the patient.

Also in Figure 1, the component compartment 14, having a top formed from the bulkhead plate 18 and the bottom from the circular base 32 pressed into the bottom of the polymer cylinder 12. The component chamber 14 houses the pumping elements, pump control module and user control panel 34. The user control panel 34 is mounted in an opening in the circumference of the polymer cylinder 12. The user control panel 34 provides a user activation switch 36 which is the sole user activating and deactivating means for the device. Also provided in the user control panel 34 is power connector 38 for providing electrical power to the device for purposes of charging internal rechargeable batteries. Visual illuminating devices are also provided on the user control panel 34 for indicating the internal battery charge levels and conditions at 40, 42, 44, and 46; and also provided is the activation status at 48.

The operation of the apparatus is easy and simple to understand as patients requiring the device are often significantly disabled. The user fills the fluid reservoir 14 with fluid to be instilled, primes the pump, if necessary, by momentarily pressing the user activation switch 36, momentarily pressing the user activation switch 36 again to deactivate the device when fluid appears at the delivery openings 43 of the stoma catheter 30, inserting the stoma catheter 30 into the patient's stoma, and momentarily pressing the user activation switch 36 once again to begin the delivery of fluid into the patient's stoma. The operation of the apparatus is thereon directed by the pump control module which maintains a safe pressure and flow rate into the patient until the instillation fluid is depleted from the fluid reservoir or directed to stop by the user momentarily pressing the user activation switch 36. The total volume of instillation fluid to be delivered into the patient is user selected by filling the fluid reservoir 14 to a preselected or prescribed graduation mark. The graduation marks 60 are etched into the transparent polymer cylinder 12 corresponding to various fluid volumes.

Referring to Figure 2, a top plan view according to the present invention, the polymer cylinder 12 surrounds the fully revealed top of the bulkhead plate 18 with the top of the pump manifold 20 protruding through and the opening in the bulkhead plate 18. Preferably, the top of the pump manifold 20 is positioned recessed slightly below the surface of the bulkhead plate 18 so as to provide a low point drain area at the bottom of the fluid reservoir. The fine sieve filter 24 is removably inserted into the top of the inlet of the pump manifold 20. The pump manifold outlet adapter 22 is shown protruding upwardly from the pump manifold 20 with the proximate end of the delivery tube 26 pressed over the adapter. Note that the elements of the pump assembly comprising not only the pump manifold 20, but also the pump motor 62, and pump drive transfer 64 located below and sealed in the component chamber and below the bulkhead plate 18. The drive transfer 64 provides a magnetically coupling with the gear pump heads located in the pump manifold 20. The user control panel 34 protrudes from the opening in the polymer cylinder into the component chamber and is fashioned to provide a watertight fitting. Note also that the fine sieve filter 24 and pump manifold 20 are positioned in the bulkhead plate 18 towards the outside circumference of the fluid reservoir so as to provide mounting space for the pump motor 62 and also to allow a user to tip the device to collect fluid over the fine sieve filter thus providing a means to reduce the residual fluid in the reservoir to a minimum.

Now in Figure 3, showing a bottom plan view according to the present invention, the circular base 32 is comprising a disc shaped element having a plurality of round recesses, typified at 52, wherein feet, being constructed of rubberized or anti-skid material, typified at 54, are inserted and secured. A battery compartment cover 56 is secured in the base 32 by threads presented around the circumference of the cover and received by threads in the base 32. Finger cut outs 66 and 68 in the cover 56 provide a grip for the user to unscrew and remove the cover 56.

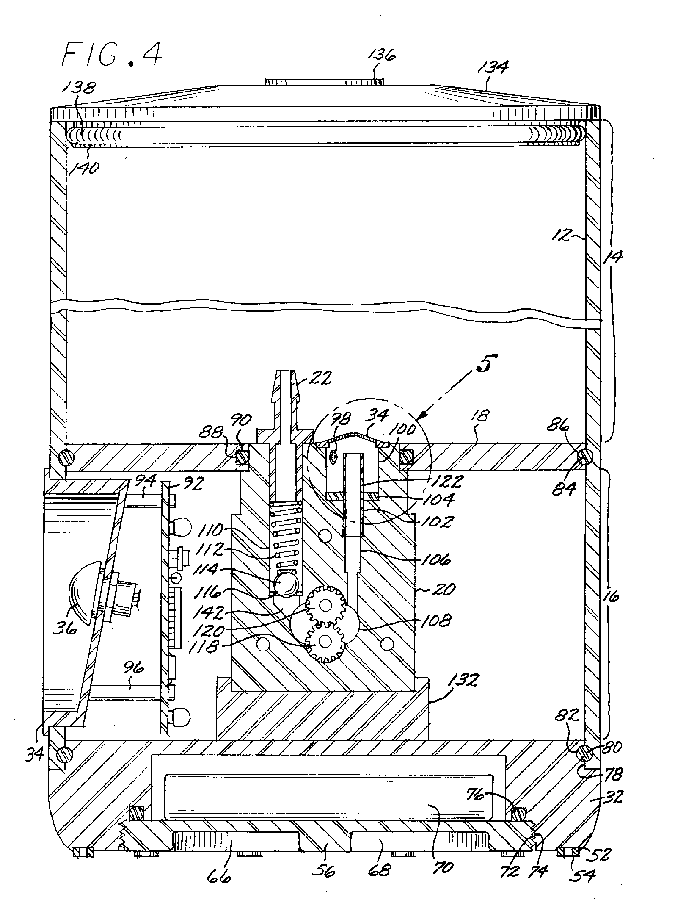

A cross-section view provided in Figure 4 reveals the internal arrangement of the apparatus. Watertightness of internal is achieved by o-ring seals between all of the major structural elements with the exception of the user control panel 34 which is secured by water resistant adhesives. The relationship between the base 32 and the cover 56 are illustrated wherein the circumference threads 72 of the cover 56 are received by the threads 74 of the base 32. Base o-ring 76 is retained in a recess of the base 32 thereby providing a watertight seal around the battery 70. The base 32 has a mounting recess 78 to fit snuggly within the polymer cylinder 12 and an o-ring recess 82 to accommodate o-ring 80 thereby providing for the polymer cylinder 12 to receive the base 32 by pressing the base 32 into the bottom opening of the polymer cylinder 12. Similarly, the bulkhead plate 18 is press fit into the inside of the polymer cylinder 12 by means of bulkhead plate o-ring 84 retained by plate outside recess 86 around the circumference of the bulkhead plate 18. Also, similarly, the top portion of the pump manifold 20 is pressed into the bulkhead plate pump o-ring 90 retained by plate inside recess 88 forming a watertight seal between the pump manifold and the bulkhead plate 18. Pump manifold mount 132 located between the pump manifold 20 and the base 32 is constructed from a resilient material being compressed upon insertion of the base 32 in the polymer cylinder to insure that the pump manifold 20 remains secured to the bulkhead plate 18.

In the preferred embodiment, the pump control module 92 is mounted to the back of the user control panel 34 by standoffs 94 and 96 within the watertight component chamber 16 as illustrated in Figure 4. The pump control module 92 is comprising a programmable processor and suitable interface electronic components to be in direct electrical communication with the user activation switch 36, the battery condition indicators 40, 42, 44 and 46, the power indicator 48, the power adapter 38, the battery 70, an optical fluid level sensor 98, and the pump motor 62. The battery condition indicators 40, 42, 44 and 46 show conditions of completely full, full, satisfactory and low while the apparatus is active or charging. The power indicator is visible when the apparatus is active. Non-rechargeable or rechargeable batteries may be utilized wherein the pump control module 92 is capable of detecting of rechargeable batteries are inserted and manages the recharging of the batteries when power is presented to the power adapter 38. The rechargeable batteries have unique connectors to facilitate detection. A specialized battery holder is provided for non-rechargeable batteries. The number of cells in the battery 70 is used to limit the maximum power to transfer to the pump thereby performing as a safety limiting mechanism dependent on the particular patient. For example, when issued to a child, a battery with fewer cells is utilized than if the apparatus were issued to an adult. The pump control module 92 is further engineered to reduce the total power transfer when non-rechargeable batteries are detected to prevent users from inadvertently applying high power as non-rechargeable batteries are intended for emergency use only.

The pump control module 92 is also operable to direct the automatic sequencing of the apparatus. When the user directs the apparatus to start pumping by pressing the user activation switch 36, the pump motor, being electric is started at a predetermined speed commensurate to satisfy the pressure and volume flow rate for the particular patient as determined by the preselected capacity of the battery and drives the gear pump drive gears 118 and 120 to rotate within the gear pump cavity 108 to draw fluid through the pump cavity inlet path 106 and out through the pump cavity outlet path 142. The intermeshed drive gears 118 and 120 are magnetically coupled to the pump drive transfer mechanism thereby isolating the fluid pathways from external contamination. As the fluid is drawn through the pump cavity 108 and out through the pump cavity outlet path 142, a back flow restrictor 110 in direct fluid communication with the pump cavity outlet path 142 is comprising a back flow restrictor valve seat 116, a valve ball 114, and a back flow flow restrictor spring 112 secured by the outlet adapter 22 positioned in the same respective order such that the fluid flow from the pump cavity 108 forces the valve ball 114 to lift from the valve seat 116 and to flow to the pump manifold outlet. The valve ball 114 re-seats by spring 112 against the valve seat 116 when the flow stops or attempts to reverse to the pump cavity. The valve ball 114 is preferably constructed of glass so as to minimize build up of deposits.

The pump control module 92 begins to monitor the optical level sensor 98 for indications of low fluid level after a 3 to 5 second delay from the beginning of activation so as to allow sufficient time to evacuate the fluid recess chamber of air. When the optical level sensor 98 indicates no fluid in the chamber, the pump control module 92 deactivates the pump motor and deactivates the power indicator 48 thereby completing the sequence. The pump control module 92 further monitors the pump motor current to indicate the fluid pressure such that the delivery of the fluid is stopped if the pressure exceeds predetermined levels for the particular patient or if an obstruction occurs preventing fluid flow.

Of particular import in the present invention, the fluid recess chamber 100, in Figure 4. The fluid recess chamber 100 having a top, width, bottom, and height forms the fluid receiving portion of the inlet of the pump manifold 20 comprising a cylindrically shaped expansion opening of the pump manifold 20 inlet being open on the top and having an outlet bore in the bottom wherein a vertical draw tube 102 is removably press fit into the bore having a length such that the top of the tube 124 is near the top of the chamber and also having a small bore 122 in the side of the tube 102 tangent to the bottom of the chamber 100 comprising seal 104. The draw tube seal 104 is constructed of resilient material formed in a disk shape having a diameter of the recess chamber and an opening to receive the vertical draw tube 102. A recess 144, as illustrated in Figure 5, around the top of the fluid recess chamber 100 receives the fine sieve filter 34. The fine sieve filter 34, the vertical draw tube 102 and the seal 104 are therefore all removable for cleaning.

The fluid recess chamber 100 is critical to the proper operation of the device and facilitates a number of significant advantages over the prior art. Firstly, as illustrated in Figure 5, the chamber permits the use of a removable fine sieve filter 34 presented to the reservoir at the pump manifold 20 inlet. Drawing fluid through a fine filter, as opposed to pushing fluid through a filter at the pump outlet, can be problematic due to air being trapped by surface tension below the filter, as indicated the air bubbles at 126, or during conditions prior to pump priming when the fluid recess chamber 100 may be empty resulting in the optical level sensor 98 detecting no fluid level in the fluid reservoir. The vertical draw tube 102, with the top 124 positioned proximate to the fine sieve filter 124, is provided to draw trapped air bubbles 126 away from regions near to the bottom of the fine sieve filter 34 when the pump motor is first started at which time the optical level sensor output is briefly disregarded by the pump control module to accomplish the procedure.

Secondly, the chamber improves the reliability of the method of detection of an empty fluid reservoir as illustrated in Figure 6. Sensing the presence of fluid utilizing mechanical means is often unreliable and prone to failure; however, optical level sensors are generally highly reliable. Using optical level sensors, however, are sensitive to ambient light levels. For example, sun light or bright lighting can falsely trigger an optical level sensor. According to the present invention, the fluid recess chamber is comprising an optical level sensor within the cylindrical wall of the chamber 100 and below the fine sieve filter 34, thus the filter shades the sensor from ambient light conditions. Because the top 124 of the vertical draw tube 102 is positioned proximate to the fine sieve filter 34, a tube drain hole 122 positioned adjacent to the bottom of the recess chamber 100 permits the chamber to drain fully reducing the fluid level below the optical level sensor 98 as the final volumes of fluid are drawn, as shown by the fluid flow at 128, into the pump. Absence of the tube drain hole 122 would result in fluid to be retained in the fluid recess chamber at levels indicated at 130.

Thirdly, incorporation of the fluid recess chamber minimizes retention of the fluid in the reservoir as the recess chamber represents the lowest point in the fluid reservoir system. Consequently the fluid reservoir must be completely empty first before the optical level sensor detects a low fluid level. As the diameter of the fluid recess chamber is small relative to the fluid reservoir, the volume of retained fluid is necessarily smaller then accomplished by other methods.

The structural elements of the apparatus may be constructed of any suitable material; however, materials contacting the instillation fluid should not leach, slough or be prone to retain deposits. Further, materials contacting the instillation fluid should be resistant to corrosion.

So as to efficiently and effectively administer an antegrade colonic enema, the pump control module is programmed, pumping mechanisms are sized, and a sufficient power supply provided to deliver fluid pressures in the range of 2 mm H2O to 500 mm H20 and fluid flow rates in the range of 50 ml/minute to 600 ml/minute as measured at the standard reference pressure and temperature conditions of 1 kPa and 0 degrees Celsius.

Returning to Figure 4, a reservoir cover 134 is fitted to the top opening of the fluid reservoir 14 for purposes of storage and transport. During transport, the stoma catheter, adapter and flexible delivery tube are stowed within the fluid reservoir 14. The user pulls a centrally position cover knob 136 upwardly relative to the cover resulting in the cover o-ring 138 to be reduced in circumference as the o-ring retainer mechanism 140 reduces in diameter responsive to the knob 136 position. The user places the cover 134 into the top of the reservoir 14, then by pressing the cover knob 136 downwardly relative to the cover, the o-ring retainer mechanism 140 expands in diameter thereby compressing the cover o-ring 138 against the inside circumference of the reservoir 14 thereby providing a snug and watertight fit suitable for storage and transport of the apparatus.

Use of the portable instillation apparatus is very simple and can be managed by a patient or caregiver with very little training. As many patients use tap water as the flushing fluid for antegrade colonic enemas, the open reservoir system provides easy filling direction from a sink faucet as well as easy access for cleaning. The graduation marks provide an easy means for selecting the total volume to be administered.

More over, the present system provides facile utilization due to: (1) minimal patient training, (2) no filling of fluid reservoir bags or other sealed containers, (3) open and bottom drawn reservoir system provides easy filling and minimal air entrainment, (4) high availability and reliability, and (5) ready portability.

In view of the foregoing, it will be seen that the several objects of the invention are achieved and other advantages are attained. Although the foregoing includes a description of the best mode contemplated for carrying out the invention, the scope of which is defined by the claims, various modifications are conceivable.

## Claims

1. An instillation apparatus for the purpose of pumping fluid into the intestinal tract through a stoma opening as presented by a patient, comprising,
a fluid reservoir (14) having a housing (12), a top and a bottom suitable for holding instillation fluid, the bottom being a bulkhead plate (18),
an internal component chamber (16) having housing, a top and a bottom wherein the chamber top is the bulkhead plate (18) of the fluid reservoir (14), the bottom having a base with a cavity for holding a battery power supply (70),
a pump assembly having an electric pump motor (62), drive transfer (64) and pump manifold (20) with the pump manifold (20) having a pump cavity, an inlet and an outlet (22), mounted from below through an opening in bottom of the fluid reservoir (14) and inside the internal component chamber (16) with the inlet in direct fluid communication with the fluid reservoir (14) and the outlet (22) protruding upwardly into the fluid reservoir (14) but not in fluid communication with the reservoir (14),
a delivery tube (126) being flexible and having a proximate and distal end with the proximate end in direct fluid communication with the pump manifold outlet (22),
a catheter (30) suitable for insertion into a patient with an intestinal stoma constructed for purposes of instilling fluid into a patient's intestines in direct fluid communication and removably attached to the distal end of the delivery tube (26),
a pump control module (92) communicatively attached to the pump motor (62) for purposes of activation, and mounted in the internal component chamber (16),
a fluid recess chamber (100) being formed as an expansion of the pump manifold (20) inlet having a top, height, wall, bottom, filter (24) in the top in direct fluid communication with the fluid reservoir (14), and a draw tube (102) positioned vertically and a top end positioned near and below the filter (24) and having a drain hole (122) tangent to the bottom of the recess chamber (100) with the draw tube (102) in direct fluid communication with the pump cavity,
a fluid level sensor (98) mounted in the fluid recess chamber (100) wall operable to communicate the presence of fluid in the chamber (100) to the pump control module (92) to which it is communicatively attached, the pump control module (92) further being in direct electrical communication with the battery power supply (70); and,
a user control panel (34) mounted proximate to the internal component chamber being in direct electrical communication with the pump control module (92).

2. The instillation apparatus of Claim 1 wherein the pump manifold (20) further comprising further a gear pump with intermeshed gears (118, 120) in the pump cavity (108) magnetically coupled to the drive transfer (64) with the pump cavity (108) being in direct fluid communication an inlet of the manifold (20) and a back flow restrictor (110), the restrictor (110) being operable to permit flow in a direction from the manifold inlet, the restrictor (110) being in further, direct fluid communication with the manifold outlet (22).

3. The instillation apparatus of Claim 1 wherein the vertical draw tube (102) is removable from the recess chamber (100) and secured in position by a resilient seal (104) having dimension of the bottom of the recess chamber (100) and an opening sized to seal against the outside circumference of the vertical draw tube (102).

4. The instillation apparatus of Claim 1 wherein the filter (24) is a removable fine sieve type filter.

5. The installation apparatus of Claim 1 wherein the fluid level sensor (98) is an optical level sensor.

6. The installation apparatus of Claim 1 wherein the housing (12) is a cylindrical polymer tube.

7. The instillation apparatus of Claim 1 wherein the user control panel (34) further comprises at least one operator control button in electrical connectivity with the pump control module (92) for activating and deactivating the pump control module (92) such that the pump control module (92) is activated by a single assertion by the operator.

8. The instillation apparatus of Claim 1 wherein the user control panel (34) further comprises battery condition indicators (40, 42, 44, 46) for completely full, full, satisfactory and low being in direct communication with the pump control module and are visible when the apparatus is active or charging.

## Patentansprüche

1. Instillationsgerät zum Pumpen einer Flüssigkeit durch eine von einem Patienten dargebotene Stomaöffnung in den Darmtrakt, umfassend:
einen Flüssigkeitsbehälter (14) mit einem Gehäuse (12), einer Oberseite und einer Unterseite, der geeignet ist, eine Instillationsflüssigkeit aufzunehmen, wobei die Unterseite eine Schottplatte (18) ist,
eine Innenkomponentenkammer (16) mit einem Gehäuse, einer Oberseite und einer Unterseite, wobei die Kammeroberseite die Schottplatte (18) des Flüssigkeitsbehälters (14) ist, wobei die Unterseite einen Boden mit einem Hohlraum zum Aufnehmen einer Batteriestromversorgung (70) aufweist,
eine Pumpenanordnung mit einem Elektropumpenmotor (62), einer Antriebsübertragung (64) und einem Pumpenverteiler (20), wobei der Pumpenverteiler (20) einen Pumpenhohlraum, einen Einlass und einen Auslass (22) aufweist und von unten durch eine Öffnung in der Unterseite des Flüssigkeitsbehälters (14) und innerhalb der Innenkomponentenkammer (16) montiert ist, wobei der Einlass in direkter Flüssigkeitskommunikation mit dem Flüssigkeitsbehälter (14) steht und der Auslass (22) nach oben in den Flüssigkeitsbehälter (14) ragt, jedoch nicht in Flüssigkeitskommunikation mit dem Behälter (14) steht,
einen Zufuhrschlauch (126), der biegsam ist und ein proximales und ein distales Ende aufweist, wobei das proximale Ende in direkter Flüssigkeitskommunikation mit dem Pumpenverteilerauslass (22) steht,
einen Katheter (30), der geeignet ist, in einen Patienten mit einem Darmstoma eingeführt zu werden, der vorgesehen ist, Flüssigkeit in den Darm eines Patienten zu instillieren, der in direkter Flüssigkeitskommunikation steht und abnehmbar mit dem distalen Ende des Zufuhrschlauchs (26) verbunden ist,
ein Pumpensteuerungsmodul (92), das zu Aktivierungszwecken in kommunikativer Weise am Pumpenmotor (62) angebracht und in der Innenkomponentenkammer (16) montiert ist,
eine Flüssigkeitsaussparungskammer (100), die als eine Erweiterung des Einlasses des Pumpenverteilers (20) ausgebildet ist, mit einer Oberseite, einer Höhe, einer Wand, einer Unterseite, einem Filter (24) in der Oberseite, der in direkter Flüssigkeitskommunikation mit dem Flüssigkeitsbehälter (14) steht, und einem vertikal positionierten Zugschlauch (102) und einem nahe und unterhalb des Filters (24) positionierten oberen Ende und mit einem tangential zur Unterseite der Aussparungskammer (100) verlaufenden Ablassloch (122), wobei der Zugschlauch (102) in direkter Flüssigkeitskommunikation mit dem Pumpenhohlraum steht,
einen in der Wand der Flüssigkeitsaussparungskammer (100) montierten Flüssigkeitsniveausensor (98), der betreibbar ist, um das Vorhandensein von Flüssigkeit in der Kammer (100) an das Pumpensteuerungsmodul (92), mit dem er in kommunikativer Weise verbunden ist, zu kommunizieren, wobei das Pumpensteuerungsmodul (92) ferner in direkter elektrischer Kommunikation mit der Batteriestromversorgung (70) steht; und
ein in der Nähe der Innenkomponentenkammer montiertes Benutzersteuerungsfeld (34), das in direkter elektrischer Kommunikation mit dem Pumpensteuerungsmodul (92) steht.

2. Instillationsgerät nach Anspruch 1, wobei der Pumpenverteiler (20) ferner eine Zahnradpumpe mit ineinandergreifenden Zahnrädern (118, 120) im Pumpenhohlraum (108) umfasst, die magnetisch mit der Antriebsübertragung (64) gekoppelt ist, wobei der Pumpenhohlraum (108) in direkter Flüssigkeitskommunikation mit einem Einlass des Verteilers (20) und einer Rückstromdrossel (110) steht, wobei die Drossel (110) betreibbar ist, um einen Strom in einer Richtung weg vom Verteilereinlass zuzulassen, wobei die Drossel (110) ferner in direkter Flüssigkeitskommunikation mit dem Verteilerauslass (22) steht.

3. Instillationsgerät nach Anspruch 1, wobei der vertikale Zugschlauch (102) von der Aussparungskammer (100) abnehmbar und in seiner Position durch eine elastische Dichtung (104) gesichert ist, welche die Größe der Unterseite der Aussparungskammer (100) und eine Öffnung aufweist, die so dimensioniert ist, dass sie gegen den Außenumfang des vertikalen Zugschlauchs (102) abdichtet.

4. Instillationsgerät nach Anspruch 1, wobei der Filter (24) ein abnehmbarer Filter von der Art eines feinen Siebs ist.

5. Instillationsgerät nach Anspruch 1, wobei der Flüssigkeitsniveausensor (98) ein optischer Niveausensor ist.

6. Instillationsgerät nach Anspruch 1, wobei das Gehäuse (12) ein zylinderförmiger Polymerschlauch ist.

7. Instillationsgerät nach Anspruch 1, wobei das Benutzersteuerungsfeld (34) ferner zumindest eine Bedienersteuerungstaste umfasst, die zum Aktivieren und Deaktivieren des Pumpensteuerungsmoduls (92) in elektrischer Verbindung mit dem Pumpensteuerungsmodul (92) steht, sodass das Pumpensteuerungsmodul (92) durch eine einzige Handlung des Bedieners aktiviert wird.

8. Instillationsgerät nach Anspruch 1, wobei das Benutzersteuerungsfeld (34) ferner Batteriezustandsindikatoren (40, 42, 44, 46) für voll geladen, geladen, ausreichend und leer umfasst, die in direkter Kommunikation mit dem Pumpensteuerungsmodul stehen und sichtbar sind, wenn sich das Gerät im Betriebs- oder Ladezustand befindet.

## Revendications

1. Appareil d'instillation afin de pomper du fluide dans le tractus intestinal par une ouverture de stomie telle que présentée par un patient, comprenant :
un réservoir de fluide (14) ayant un boîtier (12), un sommet et un fond appropriés pour contenir le fluide d'instillation, le fond étant une plaque formant cloison (18),
une chambre de composant interne (16) ayant le boîtier, un sommet et un fond, dans lequel le sommet de la chambre est la plaque formant cloison (18) du réservoir de fluide (14), le fond ayant une base avec une cavité pour contenir une alimentation de batterie (70),
un ensemble de pompe ayant un moteur de pompe électrique (62), un transfert d'entraînement (64) et un collecteur de pompe (20), avec le collecteur de pompe (20) qui a une cavité de pompe, une entrée et une sortie (22), montées par dessous par une ouverture au fond du réservoir de fluide (14) et à l'intérieur de la chambre de composant interne (16) avec l'entrée en communication de fluide directe avec le réservoir de fluide (14) et la sortie (22) qui fait saillie vers le haut dans le réservoir de fluide (14) mais pas en communication de fluide avec le réservoir (14),
un tube d'administration (126) qui est flexible et ayant une extrémité proximale et une extrémité distale, avec l'extrémité proximale en communication de fluide directe avec la sortie (22) du collecteur de pompe,
un cathéter (30) approprié pour l'insertion dans un patient avec une stomie intestinal réalisée afin d'instiller le fluide dans les intestins d'un patient en communication de fluide directe et fixé de manière amovible à l'extrémité distale du tube d'administration (26),
un module de commande de pompe (92) fixé en communication au moteur de pompe (62) à des fins d'activation, et monté dans la chambre de composant interne (16),
une chambre formant évidement de fluide (100) étant formée comme une expansion de l'entrée du collecteur de pompe (20) ayant un sommet, une hauteur, une paroi, un fond, un filtre (24) dans le sommet en communication de fluide directe avec le réservoir de fluide (14) et un tube d'aspiration (102) positionné verticalement et une extrémité supérieure positionnée à proximité de et au-dessous du filtre (24) et ayant un trou de drain (122) tangent par rapport au fond de la chambre formant évidement (100), avec le tube d'aspiration (102) en communication de fluide directe avec la cavité de pompe,
un capteur de niveau de fluide (98) monté dans la paroi de la chambre formant évidement de fluide (100) pouvant fonctionner pour communiquer la présence du fluide dans la chambre (100) au module de commande de pompe (92) auquel il est fixé par communication, le module de commande de pompe (92) étant en outre en communication électrique directe avec l'alimentation de batterie (70) ; et
un panneau de commande utilisateur (34) monté à proximité de la chambre de composant interne qui est en communication électrique directe avec le module de commande de pompe (92).

2. Appareil d'instillation selon la revendication 1, dans lequel le collecteur de pompe (20) comprend en outre une autre pompe à engrenages avec des engrenages (118, 120) mutuellement engrenés dans la cavité de pompe (108) magnétiquement couplée au transfert d'entraînement (64), avec la cavité de pompe (108) qui est en communication de fluide directe avec une entrée du collecteur (20) et un limiteur de refoulement (110), le limiteur (110) pouvant fonctionner pour permettre l'écoulement dans une direction à partir de l'entrée du collecteur, le limiteur (110) étant en outre en communication de fluide directe avec la sortie (22) du collecteur.

3. Appareil d'instillation selon la revendication 1, dans lequel le tube d'aspiration (102) vertical est amovible de la chambre formant évidement (100) et fixé en position par un joint d'étanchéité élastique (104) ayant la dimension du fond de la chambre formant évidement (100) et une ouverture dimensionnée pour réaliser l'étanchéité contre la circonférence extérieure du tube d'aspiration (102) vertical.

4. Appareil d'instillation selon la revendication 1, dans lequel le filtre (24) est un filtre de type à tamis fin amovible.

5. Appareil d'instillation selon la revendication 1, dans lequel le capteur de niveau de fluide (98) est un capteur de niveau optique.

6. Appareil d'instillation selon la revendication 1, dans lequel le boîtier (12) est un tube polymère cylindrique.

7. Appareil d'instillation selon la revendication 1, dans lequel le panneau de commande utilisateur (34) comprend en outre au moins un bouton de commande d'opérateur raccordé électriquement au module de commande de pompe (92) pour activer et désactiver le module de commande de pompe (92) de sorte que le module de commande de pompe (92) est activé par une seule assertion par l'opérateur.

8. Appareil d'instillation selon la revendication 1, dans lequel le panneau de commande utilisateur (34) comprend en outre des indicateurs d'état de batterie (40, 42, 44, 46) pour indiquer un niveau complètement plein, un niveau plein, un niveau satisfaisant et un niveau faible, qui sont en communication directe avec le module de commande de pompe et sont visibles lorsque l'appareil fonctionne ou est en charge.
